(19) Europäisches Patentamt
European Patent Office
Office européen des brevets

(11) EP 3 945 316 A2

(12) EUROPEAN PATENT APPLICATION

(43) Date of publication:
02.02.2022 Bulletin 2022/05

(51) International Patent Classification (IPC):
G01N 33/204 (2019.01)

(21) Application number: 21157543.6

(22) Date of filing: 17.02.2021

(52) Cooperative Patent Classification (CPC):
G01N 33/204

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 31.07.2020 KR 20200095746

(71) Applicant: MACTEC Corp.
Daejeon 34057 (KR)

(72) Inventors:
• KIM, Young Suk
34070 Daejeon (KR)
• KIM, Sung Soo
34010 Daejeon (KR)

(74) Representative: Isarpatent
Patent- und Rechtsanwälte Barth
Charles Hassa Peckmann & Partner mbB
Friedrichstrasse 31
80801 München (DE)

(54) **METHOD OF DIAGNOSING THE LIFETIME OF A STRUCTURE AND SYSTEM FOR DIAGNOSING THE SAME**

(57) The present invention relates to a method of diagnosing the lifetime of a structure and a system for diagnosing the same. The method of diagnosing the lifetime of a structure may comprise a step for preparing a structure to be measured; a step for measuring an amount of exothermic or endothermic heat of the structure; a step for measuring an amount of entropy decrease of the structure by using the measured amount of exothermic or endothermic heat; and a step for diagnosing the remaining lifetime of the structure from the measured amount of entropy decrease.

*FIG. 17*

EP 3 945 316 A2

**Description**

CROSS-REFERENCES TO RELATED APPLICATION

The present application claims the benefit of Korean application No. 10-2020-0095746, filed on July 31, 2020, which is herein incorporated by reference in its entirety.

BACKGROUND OF THE INVENTION

1. Field

The present invention relates to life diagnosis technology, and more particularly, to a method of diagnosing the lifetime of a structure and system for diagnosing the same.

2. Description of the Related Art

As all the structures including infrastructure and utilities existing on earth age with increasing operating time, their catastrophic failures become increasingly possible. Therefore, it is very important to determine the remaining lifetime of an aged structure and assess its sustainability in view of public safety and economic development. Right now, the nuclear industry has currently been trying to extend the lifetime of nuclear power plants from 40 to 60 years in Korea, and from 60 to 80 years, especially in the United States, among foreign countries. To this end, however, the first thing to be addressed is to assure the sustainability of the reactor core structures such as pressure vessels, pipings, steam generators or pressurizers, and electric cables being used in nuclear power plants.

In addition, since the structures of thermal power generation plants, petrochemical facilities, and district heating plants operate at high temperatures, the technologies for diagnosing the sustainability and lifetime of the structures being exposed at high temperatures are also desperately needed in the field of these industries.

It is generally accepted that the degree of aging of the structures described above is primarily determined by external factors such as external stress, fatigue, creep, environmental effects such as corrosion, oxidation, erosion and hydrogen embrittlement, and initial material parameters. Nevertheless, since the aging phenomena of the structures themselves have not yet been clearly understood, the current methods of assessing the sustainability and lifetime of the structures that have been established based on the current knowledge that the aging of the structures is determined primarily by external factors may be too incomplete and defective to reliably assess their sustainability and lifetime.

As an alternative to the life diagnosis, instead, non-destructive examination technologies are widely being used to detect defects existing in a structure and to measure their sizes for the safety of the structure. However, the non-destructive examination technologies cannot specify the cause of the defects, leading to a difficulty in diagnosing the sustainability and the accurately remaining lifetime of the aged structure. In particular, current non-destructive examination techniques have technical limitations in identifying fine defects below 0.25 mm in size which can critically affect the lifetime of the structures.

There have been many catastrophic failures of the aged structures due to inaccurate life assessment, which tend to become more and more with increasing operating time.

Therefore, there is a strong incentive for a life diagnosis method and a related system that may accurately diagnose the remaining lifetime of a structure.

SUMMARY OF THE INVENTION

The aim of the present invention is to provide a method of accurately diagnosing the lifetime of a structure.

In addition, the another aim of the present invention is to provide a system for accurately diagnosing the lifetime of the structure.

According to an embodiment of the present invention, there may be provided a method of diagnosing the lifetime of a structure comprising; a step for preparing a structure to be measured; a step for measuring the amount of exothermic or endothermic heat of the structure; a step for determining the amount of entropy decrease of the structure from the measured amount of heat; and a step for diagnosing the remaining lifetime of the structure from the measured amount of entropy decrease. The step for preparing a structure to be measured may comprise a step for selecting the most degraded part of the structure to be measured. The step for diagnosing the remaining lifetime of the structure may include a relationship where a rate of entropy decrease between an arbitrary time and the end of life is determined by an activation energy for entropy decrease and a difference between the operating temperature and the peak temperature where the maximum amount of heat is released. Here, the amount of entropy decrease at an arbitrary time is determined by an amount of exothermic or endothermic heat at the corresponding time. The relationship for diagnosing the remaining lifetime, $t_L$, may be defined as

$$t_L = k(H_L/Hi)[(T_P-T)/T_P]^2 \exp[+Q_{\Delta S}/R(1/T-1/T_P)].$$

Here, k is an intrinsic constant, R is the gas constant, Tp is the peak temperature where the maximum amount of heat is released, $Q_{\Delta S}$ is an activation energy for entropy decrease, T is an operating temperature, $H_L$ is an amount of exothermic or endothermic heat at the end of life, and $H_i$ is an amount of exothermic or endothermic heat determined at an arbitrary time. The activation energy for entropy decrease, $Q_{\Delta S}$ may be determined by the Kissinger method where the peak temperature where the maximum amount of heat is evolved is plotted as a function of heating rate.

A step for measuring the actual compressive stress of the structure; and a step for correcting the predicted remaining lifetime based on a difference between the measured compressive stress value, and the predicted compressive stress value obtaining from the measured amount of exothermic or endothermic heat may be further performed. The amount of exothermic or endothermic heat of the structure can be determined using differential scanning calorimetry (DSC), differential thermal analysis (DTA), thermogravimetric analysis (TGA), thermomechanical analysis (TMA), dynamic mechanical analysis(DMA), or a combination thereof.

According to another embodiment of the present invention, there may be provided a method of diagnosing the lifetime of a structure comprising a step for preparing a structure to be measured; a step for measuring the magnitude of compressive stress of the structure; a step for measuring an amount of entropy decrease of the structure from the measured magnitude of compressive stress; and a step for diagnosing the remaining lifetime of the structure from the measured amount of entropy decrease. The step for diagnosing the remaining lifetime of the structure by measuring an amount of entropy decrease may include a relationship where a rate of entropy decrease between an arbitrary time and the end of life is determined by an activation energy for entropy decrease and a difference between the operating temperature and the peak temperature where the maximum amount of heat is released. Here, an amount of entropy decrease at any time is represented by the magnitude of compressive stress measured at that time, and the relationship for diagnosing the remaining lifetime, $t_L$, is defined as

$$t_L = k(\sigma_L/\sigma_i)[(T_P-T)/T_P]^2 \exp[+Q_{\Delta S}/R*(1/T-1/T_P)],$$

wherein, k is an intrinsic constant, R is the gas constant, Tp is the peak temperature where the maximum amount of heat is released, $Q_{\Delta S}$ is an activation energy for entropy decrease, T is an operating temperature, $\sigma_L$ is a magnitude of compressive stress at the end of life, and $\sigma_i$ is a magnitude of compressive stress measured at an arbitrary time.

According to yet another embodiment of the present invention, there may be provided a method of diagnosing the lifetime of a structure comprising; a step for preparing a structure to be measured; a step for measuring the amount of lattice contraction of the structure; a step for measuring an amount of entropy decrease of the structure from the measured amount of lattice contraction; and a step for diagnosing the remaining lifetime of the structure from the measured amount of entropy decrease. The step for diagnosing the remaining lifetime of the structure may include a relationship where a rate of entropy decrease between an arbitrary time and the end of life is determined by an activation energy for entropy decrease and a difference between the operating temperature and the peak temperature where the maximum amount of heat is released. Here, an amount of entropy decrease at any time is obtained from the amount of lattice contraction measured at that time, and the relationship for diagnosing the remaining lifetime, $t_L$, is defined as

$$t_L = k(\Delta a_L/\Delta a_i)[(T_P-T)/T_P]^2 \exp[+Q_{\Delta S}/R*(1/T-1/T_P)],$$

wherein, k is an intrinsic constant, R is the gas constant, Tp is the peak temperature where the maximum amount of heat is released, $Q_{\Delta S}$ is an activation energy for entropy decrease, T is an operating temperature, $\Delta a_L$ is an amount of lattice contraction at the end of life of the structure, and $\Delta a_i$ is an amount of lattice contraction of the structure at an arbitrary time.

According to another embodiment of the present invention, there may be provided a method of diagnosing the lifetime of a structure comprising; a step for preparing a structure to be measured; a step for measuring the physical properties of the structure; a step for measuring an amount of entropy decrease of the structure by measuring changes in physical properties; and a step for diagnosing the remaining lifetime of the structure by measuring the amount of entropy decrease from the measured changes in physical properties, wherein the physical properties include hardness, thermoelectric power, electrical resistance, Barkuhausen noise amplitude, shear modulus, elastic modulus or Young's modulus, or a combination thereof of the structure.

According to another embodiment of the present invention, there may be provided a life diagnosis system of a structure comprising: measuring devices which determine an amount of exothermic or endothermic heat of the structure, a magnitude of compressive stress of the structure, an amount of lattice contraction of the structure, or changes in physical properties of the structure; and an electronic device for measuring an amount of entropy decrease of the structure from the measured values such as the amount of exothermic or endothermic heat, the magnitude of compressive stress, the amount of lattice contraction, or the changes in physical properties, and diagnosing the remaining lifetime of the structure from the measured amount of entropy decrease. The physical properties include hardness, thermoelectric power, electrical resistance, Barkuhausen noise amplitude, shear modulus, elastic modulus or Young's modulus, or a combination thereof of the structure.

The step for diagnosing the remaining lifetime of the structure from the measured amount of entropy decrease may include a relationship where a rate of entropy decrease between an arbitrary time and the end of life is determined by an activation energy for entropy decrease and a difference between the operating temperature and the peak temperature where the maximum amount of heat is released.

The processor may use a relationship where a rate of entropy decrease between an arbitrary time and the end of life is determined by an activation energy for entropy decrease, a difference between the operating temperature and the peak temperature where the maximum amount of heat is released, and amounts of entropy decrease measured at both an arbitrary time and the end of life. Here, the amounts of entropy decrease measured at both an arbitrary time and the end of life are represented by the amount of exothermic or endothermic heat, the amount of lattice contraction, the magnitude of compressive stress, the changes in physical properties such as hardness, thermoelectric power, electrical resistance, Barkuhausen noise amplitude, shear modulus, elastic modulus or Young's modulus, or a combination thereof of the structure, at both an arbitrary time and the end of life. The above relationship may be defined as

$$t_L=k(H_L/Hi)[(T_P-T)/T_P]^2 exp[+Q_{\Delta S}/R(1/T-1/T_P)],$$

$$t_L=k(\sigma_L/\sigma_i)[(T_P-T)/T_P]^2 exp[+Q_{\Delta S}/R*(1/T- 1/T_P)],$$

$$t_L=k(\Delta a_L/\Delta a_i)[(T_P-T)/T_P]^2 exp[+Q_{\Delta S}/R*(1/T-1/T_P)],$$

or

$$t_L=k(\Delta \Pi_L/\Delta \Pi_i)[(T_P- T)/T_P]^2 exp[+Q_{\Delta S}/R*(1/T-1/T_P)].$$

Here, k is an intrinsic constant, R is the gas constant, Tp is the peak temperature where the maximum amount of heat is released, $Q_{\Delta S}$ is an activation energy for entropy decrease, T is an operating temperature, $H_L$ is an amount of exothermic or endothermic heat at the end of life of the structure, and $H_i$ is an amount of exothermic or endothermic heat measured at an arbitrary time, $\sigma_L$ is a magnitude of compressive stress at the end of life of the structure, $\sigma_i$ is a magnitude of compressive stress measured at an arbitrary time, $\Delta a_L$ is an amount of lattice contraction at the end of life of the structure, $\Delta a_i$ is an amount of lattice contraction of the structure at an arbitrary time, $\Delta \Pi_L$ is an amount of changes in physical properties at the end of life of the structure, and $\Delta \Pi_i$ is an amount of changes in physical properties at an arbitrary time. The activation energy for entropy decrease, $Q_{\Delta S}$ may be determined by the Kissinger method where the peak temperature where the maximum amount of heat is evolved is plotted as a function of heating rate. In addition, the above-described processor can correct the remaining lifetime diagnosed as shown above by comparing a difference between the directly measured compressive stress value and the predicted compressive value obtaining from the measured amount of exothermic or endothermic heat.

According to an embodiment of the present invention, an amount of entropy decrease of a structure may be determined from the amount of exothermic or endothermic heat of the structure, and the remaining lifetime of the structure may be diagnosed from the measured amount of entropy decrease. Therefore, it is possible to provide a method of diagnosing the lifetime of the structure in a reliable, quick and accurate non-destructive way.

Further, according to another embodiment of the present invention, a system for diagnosing the lifetime of a structure having the above-described advantages may be provided.

BRIEF DESCRIPTION OF THE DRAWINGS

FIG. 1A and FIG. 1B are the flowcharts illustrating a method of diagnosing the lifetime of a structure according to the embodiments of the present invention, respectively.

FIG. 2 is a block diagram of a system for diagnosing the lifetime of a structure according to the embodiments of the present invention.

FIG. 3 is a diagram illustrating the cracking of muds occurring during a drought.

FIG. 4 is a graph showing the specific heats of water-quenched and furnace-cooled Alloy 600 where heat was evolved only from the water-quenched one but not from the furnace-cooled one.

FIG. 5 is a graph showing the lattice contraction behavior of the water-quenched and furnace-cooled Alloy 600 specimens during aging at 400°C.

FIG. 6A and FIG. 6B are graphs showing the relationship between performance quality and exothermic heat of a plastic printing ink.

FIG. 7A and Fig. 7B are graphs showing an increase in hardness of a 316 stainless steel during aging at 400°C and an increase in thermal conductivity of Alloy 690 upon aging at 475°C, respectively.

FIG. 8A and FIG. 8B are graphs showing the thermoelectric power and the maximum amplitude of Barkuhausen noise rms signal of a carbon steel containing 130 ppm carbon upon aging at 100°C, respectively.

FIG. 9 is a graph showing the specific heats of a water-quenched carbon steel after a homogenization treatment in an austenite region.

FIG. 10A is a graph showing a change in electrical resistance of a 30% cold-worked 316L stainless steel upon aging at 400 °C, and FIG. 10B is a graph showing changes in electrical resistance of the Fe-Ni-C iron-based alloy with aging temperature for 3 hours and with carbon concentration.

FIG. 11 is a graph showing DSC (differential scanning calorimetry) thermograms of unaged and aged Al-3.2Mg-0.18Cu alloy after a solution treatment.

FIG. 12 is a graph showing changes in elastic modulus of a Zr-2.5Nb specimen containing 86 ppm hydrogen upon hydride dissolution during heating and upon hydride precipitation during cooling.

FIG. 13 is a graph showing the specific heat of Zircaloy-2 containing 106 ppm hydrogen upon cooling at a rate of 10 °C/min.

FIG. 14 is a graph showing an increase in the amounts of exothermic and endothermic heat of a XLPE (cross-linked polyethylene) plastic with the degree of aging which is used as a cable insulation material in nuclear power plants.

FIG. 15 is a graph showing the activation energy for entropy decrease (or ordering) of Alloy 600 with carbon concentration.

FIG. 16 is a graph showing the cumulative number of stress corrosion cracking (SCC) failures with operating time for Alloy 600 components being used in nuclear power plants.

FIG. 17 is a graph comparing the predicted lifetime of Alloy 600 according to the embodiments of the present invention with the measured lifetime of Alloy 600 that was obtained from SCC failures not only in nuclear power plants but also in laboratory environments.

DETAILED DESCRIPTION OF THE INVENTION

Hereinafter, the preferred embodiments of the present invention will be described in detail with reference to the accompanying drawings.

The embodiments of the present invention are provided to more fully describe the present invention to those having a common knowledge in the related art, and the following embodiments may be modified into various other forms, and the scope of the present invention is not limited to the following embodiments. Rather, these embodiments are provided to explain the present invention more clearly and completely, and to fully convey the spirit of the present invention to those skilled in the art.

In addition, in the following drawings, a thickness or a size of each layer is exaggerated for convenience and clarity of description, and the same reference numerals in the drawings refer to the same elements. As used herein, the term, "and/or" includes any one and all combinations of one or more of the listed items.

The terminology used herein is used to describe a specific embodiment and is not intended to limit the present invention. As used herein, a singular form may include plural forms unless the context clearly indicates otherwise. Also, as used herein, the term such as "comprise" and/or "comprising" specifies the mentioned shapes, numbers, steps, actions, members, elements and/or the presence of these groups, and does not exclude the presence or addition of one or more other shapes, numbers, actions, members, elements and/or presence or addition of groups.

Although the terms, such as the first, the second, etc. are used herein to describe various members, components, regions, layers and/or portions, it is obvious that these members, components, regions, layers and/or portions are not defined by these terms. These terms are only used to distinguish one member, component, region, layer or portion from another region, layer or portion. Accordingly, the first member, component, region, layer or portion as described below may refer to the second member, component, region, layer or portion without departing from the teachings of the present invention.

Hereinafter, embodiments of the present invention will be described with reference to the drawings schematically showing ideal embodiments of the present invention. In the drawings, for example, the size and shape of members may be exaggerated for convenience and clarity of description, and in actual implementation, variations of the illustrated shape may be expected. Accordingly, embodiments of the present invention should not be construed as being limited to the specific shape of the region shown in this specification.

In order to explain the embodiments of the present invention, the second law of thermodynamics may need to be considered. According to the second law of thermodynamics, the amount of entropy change $\Delta S$ is defined as the amount of heat $\Delta H$ (or Q) released from or absorbed into a system divided by a temperature T of the system by the following <Equation 1>.

$$[\text{Equation 1}] \; \Delta S = \Delta H / T \; (J/gK)$$

Here, J represents energy in joule and K is the unit of absolute temperature.

When heat is released from the system, the entropy of the system decreases by $-\Delta H/T$, and when heat is absorbed into the system, the entropy of the system increases by $+\Delta H/T$. For example, if heat transfers from a high temperature object to a cold surrounding by $\Delta H$, the entropy of the object decreases by $-\Delta H/T_{hot}$, but the entropy of the surrounding increases by $+\Delta H/T_{cold}$. The sum of entropy changes in the object and its surroundings is expressed by Equation 2 as below.

$$[\text{Equation 2}] \; \Delta S = -\Delta H/T_{hot} + \Delta H/T_{cold}$$

In <Equation 2>, the sum of entropy changes $\Delta S$ of <Equation 2> is greater than 0 because of $T_{hot} > T_{cold}$. Therefore, the transfer of heat from a hot object to a cold environment may be considered as an entropy increase.

Similarly, the entropy of a structure may decrease or increase due to heat transfer between the structure and the surrounding, leading to a physical change in the structure. Specifically, an entropy decrease may induce a compressive stress in the structure, causing a volume shrinkage of the structure. Conversely, an entropy increase may induce a tensile stress in the structure, resulting in a volume expansion of the structure. A typical example in which the tensile stress is generated by the entropy increase is a volume expansion by around 2400 times occurring upon the transformation of water to water vapor. Conversely, a representative example in which a compressive stress is generated by the entropy decrease is a solidification shrinkage occurring when a liquid metal is solidified. For a liquid metal to become solid, heat must be released from the liquid metal, leading it to be cooled. Accordingly, the entropy of the liquid metal decreases by an amount of heat released as shown in Eq. 1, causing a compressive stress to be induced in the liquid metal, and thereby a solidification shrinkage.

The present invention, as described above, includes embodiments of a method and a system for diagnosing the lifetime of a structure by measuring changes in the amount of exothermic or endothermic heat of the structure and thereby an amount of entropy decrease of the structure, regardless of whether it consists of organic or inorganic or composite materials. Hereinafter, the embodiments of the present invention describe a method of diagnosing the remaining lifetime of a structure by measuring an amount of entropy decrease of the structure.

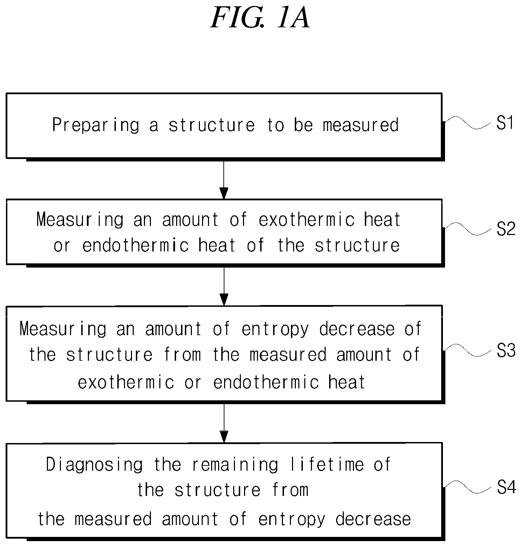

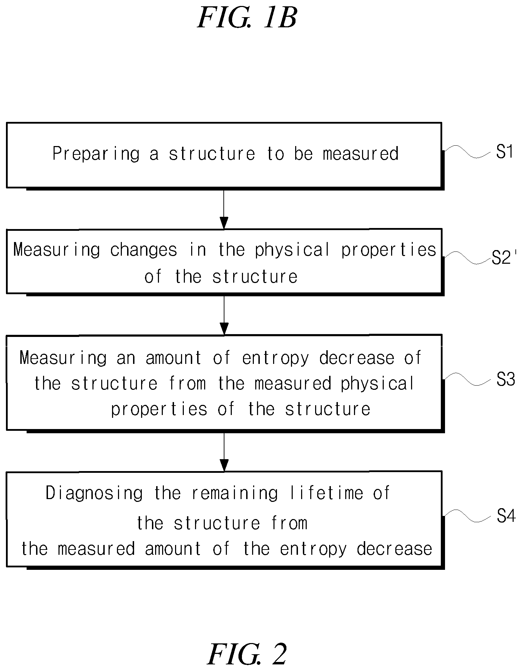

FIG. 1A and FIG. 1B are flowcharts illustrating a method of diagnosing the lifetime of a structure according to the embodiments of the present invention, respectively.

Referring to FIG. 1A, the method of diagnosing the lifetime of a structure may include a step S 1 for preparing a structure to be measured, a step S2 for measuring an amount of exothermic or endothermic heat of the structure to be measured, and a step S3 for measuring an amount of entropy decrease of the structure by using the measured amount of exothermic or endothermic heat, and a step S4 for diagnosing the remaining lifetime of the structure from the measured amount of entropy decrease. An entropy decrease of the structure is not an external factor but an internal factor, and the above-described entropy decrease, $\Delta S$, may be defined as an amount of exothermic or endothermic heat, $\Delta H$ divided by a temperature T for the structure to be measured.

In one embodiment, the step S4 for diagnosing the remaining lifetime of the structure to be measured may include a relationship describing changes in an amount of entropy decrease or in a magnitude of compressive stress between the end of life and an arbitrary time. Specifically, as the unit of heat expressed in J/mol or J/g represents an energy density, it may be converted into $J/m^3$ by multiplying the energy density expressed in J/g with the density of the structure $(g/m^3)$, which corresponds to a stress in MPa. Therefore, the measured amount of heat (J/g or J/mol) may represent the stress generated in the structure. If an amount of exothermic or endothermic heat is measured at the end of life of the structure to be measured, it is possible to know a magnitude or level of compressive stress $\sigma_L$, more specifically the level of compressive stress accumulated in the structure that corresponds to the amount of exothermic or endothermic heat. Therefore, assuming that the amount of heat measured at an arbitrary time is defined as $H_i$, the compressive stress built inside the structure, termed $\sigma_i$, can be determined from $H_i$. The time required for the internally compressive stress $\sigma_i$ of the structure to increase to $\sigma_L$ may be determined by a rate of entropy decrease or an ordering rate OR. The rate of entropy decrease or OR (ordering rate) may be defined by Equation 3 below.

[Equation 3]

$$OR = k[T_P/(T_P-T)]^2\exp[-Q_{\Delta S}/R*(1/T-1/T_P)]$$

Here, Tp is the peak temperature where the maximum amount of heat is released, R is the gas constant, T is an operating temperature, $Q_{\Delta S}$ is an activation energy for entropy decrease, and k is a constant.

Referring to the following <Equation 4> the time until the internally compressive stress $\sigma_i$ increases to $\sigma_L$ at a rate of entropy decrease may be defined as the lifetime $t_L$ of the structure, which can be described in <Equation 5 > or <Equation 6> listed below.

$$[\text{Equation 4}] \quad \sigma_L=k[T_P/(T_P-T)]^2\exp[-Q_{\Delta S}/R*(1/T-1/T_P)]t_L*\sigma_i$$

$$[\text{Equation 5}] \quad t_L=k(\sigma_L/\sigma_i)[(T_P-T)/T_P]^2\exp[+Q_{\Delta S}/R*(1/T-1/T_P)]$$

$$[\text{Equation 6}] \quad t_L=k(H_L/Hi)[(T_P-T)/T_P]^2\exp[+Q_{\Delta S}/R(1/T-1/T_P)]$$

Here, k is an intrinsic constant, R is the gas constant, Tp is the peak temperature where the maximum amount of heat is released, $Q_{\Delta S}$ is an activation energy for entropy decrease, T is an operating temperature, $H_L$ is an amount of exothermic or endothermic heat at the end of life, and $\sigma_L$ is a magnitude of compressive stress at the end of life, $H_i$ is an amount of exothermic or endothermic heat at an arbitrary time, and $\sigma_i$ is a magnitude of compressive stress at an arbitrary time. The activation energy for entropy decrease, $Q_{\Delta S}$ may be determined by the Kissinger method where the peak temperature where the maximum amount of heat is released is plotted as a function of heating rate.

Referring to <Equation 5> and <Equation 6>, the remaining lifetime of a structure may be determined according to the peak temperature Tp where the maximum amount of heat is released, an activation energy for entropy decrease, $Q_{\Delta S}$, a difference between $T_P$ and T, the amount of exothermic or endothermic heat $H_L$ or a magnitude of compressive stress $\sigma_L$ at the end of life. Specifically, the higher the $H_L$ or $\sigma_L$ measured at the end of life, the longer the lifetime of the aged structure. Conversely, the higher the operating temperature (T) and the lower the peak temperature ($T_P$), the activation energy for entropy decrease ($Q_{\Delta S}$), and the amount of exothermic or endothermic heat at the end of life ($H_L$), the shorter the lifetime of the structure.

The increasing amount of entropy decrease leads to an increase in compressive stress of a structure which triggers lattice contractions in the structure. An amount of lattice contraction in the structure may be analyzed using X-ray and neutron diffraction, from which the compressive stress of the structure may be determined. The lifetime of the structure $t_L$ may be defined as [Equation 7], based on an amount of lattice contraction determined by X-ray or neutron diffraction.

$$[\text{Equation 7}] \quad t_L=k(\Delta a_L/\Delta a_i)[(T_P-T)/T_P]^2\exp[+Q_{\Delta S}/R*(1/T-1/T_P)]$$

Here, k is an intrinsic constant, R is the gas constant, Tp is the peak temperature where the maximum amount of heat is released, $Q_{\Delta S}$ is an activation energy for entropy decrease, T is an operating temperature, $\Delta a_L$ is an amount of lattice contraction at the end of life of the structure, and $\Delta a_i$ is an amount of lattice contraction at an arbitrary time.

The method may further comprise a step for actually measuring the compressive stress of the structure, and a step for correcting a predicted remaining lifetime based on a difference between the measured compressive stress value and the predicted compressive value from the measured amount of exothermic or endothermic heat. Through the correction of the predicted remaining lifetime by using the measured and predicted values of compressive stress, it is possible to more accurately diagnose the remaining lifetime of the structure.

In one embodiment, the amount of exothermic or endothermic heat may be measured by DSC, DTA, TGA, TMA, DMA, or combination thereof. However, the present invention is not limited to these measurement methods of an exothermic or endothermic heat. Preferably, the measurement of the exothermic or endothermic heat may be performed by the DSC.

As described above, an amount of entropy decrease may be determined from changes in an amount of exothermic or endothermic heat of the structure as well as changes in its physical properties as shown in FIG. 1B.

Referring to FIG. 1B, the method of diagnosing the lifetime of a structure may include a step S1 for preparing a structure to be measured, a step S2' for measuring changes in physical properties of the structure, and a step S3 for determining an amount of entropy decrease of the structure from the measured changes in physical properties, and a step S4 for diagnosing the remaining lifetime of the structure from the measured amount of entropy decrease. The description of steps S1, S3, and S4 of FIG. 1B may refer to the description of FIG. 1A if there are no contradictions.

The lifetime of the structure $t_L$ may be defined as [Equation 8], based on changes in physical properties of the structure determined by measuring devices.

$$[\text{Equation 8}]\ t_L = k(\Delta\Pi_L/\Delta\Pi_i)[(T_P-T)/T_P]^2 \exp[+Q_{\Delta S}/R*(1/T-1/T_P)].$$

Here, k is an intrinsic constant, R is the gas constant, Tp is the peak temperature where the maximum amount of heat is released, $Q_{\Delta S}$ is an activation energy for entropy decrease, T is an operating temperature, $\Delta\Pi_L$ is an amount of changes in physical properties at the end of life of the structure, and $\Delta\Pi_i$ is an amount of changes in physical properties at an arbitrary time.

In one embodiment, the physical properties may include hardness, thermoelectric power, electrical resistance, Barkuhausen noise amplitude, shear modulus, elastic modulus (Young's modulus) of the structure, or a combination thereof. However, the present invention is not limited to these physical properties. Any physical property that may determine the amount of entropy decrease of the structure may be considered and applied. Specifically, since the entropy decrease may cause changes in physical properties such as hardness, thermoelectric power, electrical resistance, Barkuhausen noise amplitude, shear modulus, and elastic modulus of the structure, the entropy decrease may be measured by tracking the changes in physical properties. For example, bread may be hardened due to water evaporation when it is exposed to ambient temperature for a long time. As a heat of evaporation is released from the bread, the entropy of the bread decreases in proportion to the amount of heat released, generating a compressive stress in the bread, which causes a hardening of the bread. In other words, the reason why the bread becomes hard upon long exposure to ambient temperature is due to the entropy decrease in the bread arising from the evaporation heat released, generating a compressive stress that leads to its hardening. This is similar to the phenomenon of mud cracking, which will be described later. The same phenomenon as the hardening of the bread may occur in all the structures so that the structures become gradually brittle with increasing operating time.

In another embodiment, an amount of entropy decrease of the structure may be computed using a combination of both the amount of exothermic or endothermic heat of the structure and the measured changes in physical properties.

As described above, the quick and accurate diagnosis of the lifetime of a structure may be done in a non-destructive manner because the lifetime of the structure is determined from the amount of entropy decrease of the structure that is obtained by directly measuring the amount of exothermic or endothermic heat of the structure to be measured.

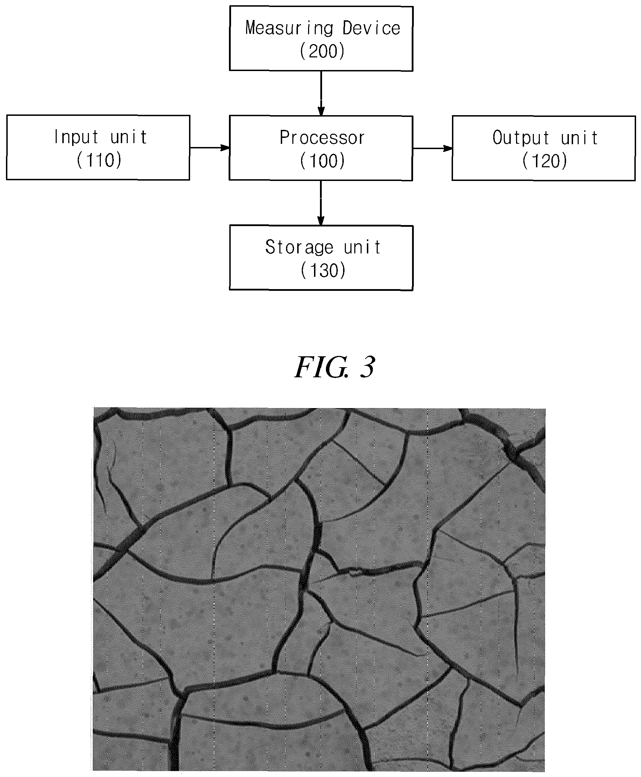

FIG. 2 is a block diagram of the life diagnosis system 10 for a structure according to the embodiments of the present invention.

Referring to FIG. 2, the life diagnosis system 10 for a structure may include a processor 100, an input unit 110, an output unit 120, a storage unit 130, and a measuring device 200. The processor 100, the input unit 110, the output unit 120, and the storage unit 130 may constitute an electronic device such as a computer or a laptop computer, and the measuring device 200 is a separate device and may be connected to the electronic device by wire or wirelessly. Alternatively, the measuring device 200 may be provided as a hardware or a software module in the electronic device. In addition, various functional blocks of the processor 100, the input unit 110, the output unit 120, and the storage unit 130 shown in FIG. 2 may include hardware elements (including circuits), software elements (including a computer code stored on a computer readable medium) or a combination of hardware and software elements.

In one embodiment, the measuring device 200 may measure the amount of exothermic or endothermic heat or the physical properties of the structure using DSC, DTA, TGA, TMA, DMA, or a combination thereof, and may provide the measured result to the processor 100. Alternatively, the measuring device 200 may measure the physical properties of the structure. In an embodiment, the physical properties may include hardness, thermoelectric power, electrical resistance, Barkuhausen noise amplitude, shear modulus, elastic modulus, or a combination of the structure to be measured. However, the present invention is not limited to these physical properties. Any physical property capable of determining the amount of entropy decrease of the structure may be applied.

The processor 100, as a CPU, may compute an amount of entropy decrease of the structure from the changes in physical properties or the amount of exothermic or endothermic heat of the structure provided by the measuring device 200, and may diagnose the remaining lifetime of the structure from the measured amount of entropy decrease. Also, the processor 100 may measure a magnitude of compressive stress of the structure. The magnitude of compressive stress may be determined by measuring an amount of entropy decrease during an operation of the structure or stress corrosion cracking (SCC) tests or by measuring the amount of lattice contraction of the structure using neutron or X-ray diffraction before and after an operation of the structure or the SCC tests.

The input unit 110 is an input device such as a keyboard, a mouse or a sensor, and may provide the input parameters to the processor 100 such as the peak temperature where the maximum heat is released, $T_P$, an activation energy for entropy decrease, $Q_{\Delta S}$, a difference between the peak temperature and the operating temperature, Tp-T, an amount of exothermic or endothermic heat at the end of life, $H_L$, a magnitude of compressive stress at the end of life, $\sigma_L$, and an amount of changes in physical properties at the end of life, $\Delta\Pi_L$, which are needed to determine the remaining lifetime of a structure to be measured.

The output unit 120 may be any suitable type of displays such as a liquid crystal display (LCD), a plasma display, or an organic light emitting diode (OLED) display. In addition, the output unit 120 may receive information on the remaining lifetime of the structure which is determined by the processor 100 and may display the information.

The storage unit 130 may further include a nonvolatile storage for a permanent storage of data and/or instructions, and may store the peak temperature, Tp, where the maximum heat is released, an activation energy for entropy decrease, $Q_{\Delta S}$, a difference between the peak temperature and the operating temperature, Tp-T, an amount of exothermic or endothermic heat at the end of life, $H_L$, a magnitude of compressive stress at the end of life, $\sigma_L$, and an amount of changes in physical properties at the end of life, $\Delta\Pi_L$, which are needed to determine the remaining lifetime of the structures to be measured. If necessary, the storage unit 130 may provide the stored parameters to the processor 100.

FIG. 3 is a diagram illustrating the cracking of muds during a drought as an example of the natural aging of a structure.

Referring to FIG. 3, when the water of the paddy mud evaporates during a drought, heat of the mud decreases due to a heat of vaporization released, causing its entropy decrease. The entropy decrease causes the internal compressive stress to incur in the mud, leading the mud to shrink and become hard. With the amount of shrinkage increasing with time, the mud fails by cracking. It is to note that the cracks are spontaneously generated in the mud, corresponding to a structure, due to the entropy decrease of the mud, generating compressive stresses in the mud and therefore its shrinkage. This is a typical example of supporting the aging law of structures where their cracking occurs spontaneously even without external factors.

In the present invention, it may be seen that the cracking of the structure is caused by compressive stresses spontaneously generated inside the structure due to an entropy decrease of the structure. Specifically, the entropy decrease of the structure occurs during its operation, which generates compressive stresses in it and thereby triggers the cracking or failures of the structure. Accordingly, in the embodiments of the present invention, the lifetime of all organic, inorganic, or composite structures thereof may be determined with the amount of entropy decrease.

In an embodiment, as the amount of entropy decrease of the structure increases, the compressive stress induced inside the structure increases, leading to the earlier cracking of the structure and thereby shortening the lifetime of the structure. In addition, since the entropy decrease occurs in all kinds of materials regardless of inorganic and organic materials, a method of diagnosing the lifetime of a structure based on the amount of entropy decrease may be universally applied to all the structures consisting of metals, ceramics, plastics, and concretes.

Experiment 1: Example of the cracking of a metal due to entropy decrease

After a homogenization treatment at about 1,025 °C for 2 hours, furnace cooling and water cooling were applied to prepare water-quenched (WQ-) and furnace-cooled (FC-) Alloy 600 steam generator tubes, and these two types of specimens were exposed to 332 °C water (e.g., high temperature water simulating the operating conditions of pressurized water reactors) for about 24,000 hours. Then, their SCC resistance was compared.

The following <Table 1> is a table showing the SCC failure rates of the WQ- and FC-Alloy 600 steam generator tube specimens.

[Table 1]

| Solution treatment conditions | Cooling rate | SCC failure rate (%) |
|---|---|---|
| 1025 °C, 2 hours | water-quenched specimen | 100 |
| | furnace-cooled specimen | 0 |

Referring to Table 1 shown above, all the WQ-Alloy 600 specimen failed by SCC cracking, while the FC-Alloy 600 specimens showed no cracking at all. In order to explain the observations in view of entropy decrease, DSC tests were performed on an Alloy 600 specimen water quenched (WQ-) and furnace-cooled (FC-) after a solution treatment at 1,095°C for 30 minutes.

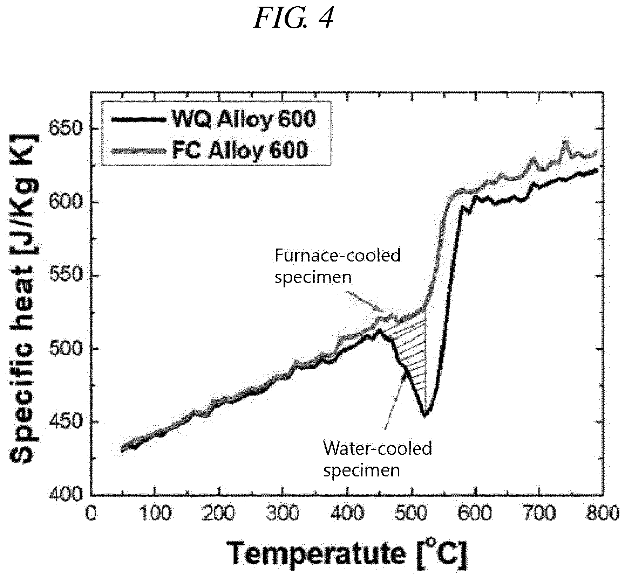

FIG. 4 is a graph showing the specific heats of water-quenched and furnace-cooled Alloy 600 where heat was evolved only from the water-quenched one but not from the furnace-cooled one.

Referring to FIG. 4, the WQ-Alloy 600 specimen showed an exothermic heat upon heating, which was not observed in the FC-Alloy 600. This result indicates that during the SCC tests at a high temperature, the water-quenched specimen releases heat, triggering an entropy decrease and thereby inducing compressive stresses. The compressive stresses induced in the matrix of the WQ-Alloy 600 cause its lattice contraction and generate tensile stresses only at grain boundaries, which caused the WQ-Alloy 600 to be more susceptible to intergranular stress corrosion cracking than the FC-Alloy 600. On the other hand, the FC-Alloy 600 that exhibited no exothermic heat upon heating will have no entropy decrease during the SCC tests, thereby inducing little compressive stresses and little lattice contraction. In order to show the validity of this claim, the WQ- and FC-Alloy 600 specimens were aged at about 400°C and their lattice contractions on the (111) plane were determined with aging time at 400°C using neutron diffraction.

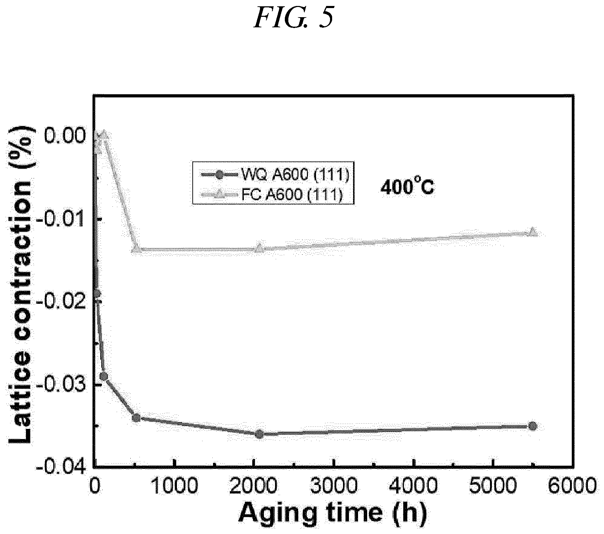

FIG. 5 is a diagram showing the lattice contraction behavior of the WQ- and FC-Alloy 600 specimens during aging treatment at 400°C.

Referring to FIG. 5, as expected, the amount of lattice contraction was much greater in the WQ-Alloy than the FC-Alloy 600. It is clearly evident that the WQ-specimen with the high amount of heat released during the heating process, as shown in FIG. 4 causes a high amount of entropy decrease during the SCC test, generating a large compressive stress and thereby a large amount of lattice contraction (FIG. 5).

Summarizing the results of Table 1 and FIG. 4 and FIG. 5, the WQ-Alloy 600 specimen with a high amount of entropy decrease due to heat evolution during SCC tests was susceptible to SCC. In contrast, the FC-Alloy 600 specimens with little entropy decrease due to little heat evolution during SCC tests showed high resistance to SCC. In summary, it should be noted that the SCC resistance of Alloy 600 is affected by the amount of entropy decrease occurring during SCC testing.

Experiment 2: Example of performance quality of a printing ink (plastic) with entropy decrease.

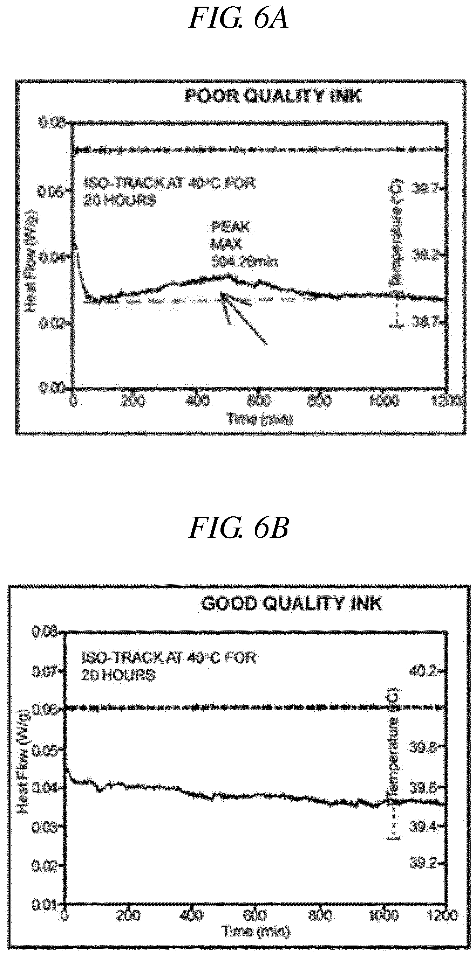

FIG. 6A and FIG. 6B show the relationship between performance quality and exothermic heat behavior of a printing ink (plastic) obtained during isothermal tests at 40°C using a DSC.

Referring to FIG. 6A, the printing ink material (plastic) exhibiting an exothermic heat showed a low-quality performance due to a rapid aging rate. However, as shown in FIG. 6B, the printing ink material with no exothermic heat showed a high-quality performance due to a slow aging rate. Specifically, it shows that a material having a large amount of exothermic heat generates a high compressive stress due to a high amount of entropy decrease, leading to a large amount of lattice contraction, which caused rapid aging and poor performance. Judging from these results, it may be seen that the higher the amount of exothermic heat, the higher the aging rate of the structure and the shorter the lifetime. Therefore, the lifetime of the structure may be determined by measuring the amount of exothermic or endothermic heat of the structure.

Experiment 3: Example of changes in physical properties of the structures due to entropy decrease

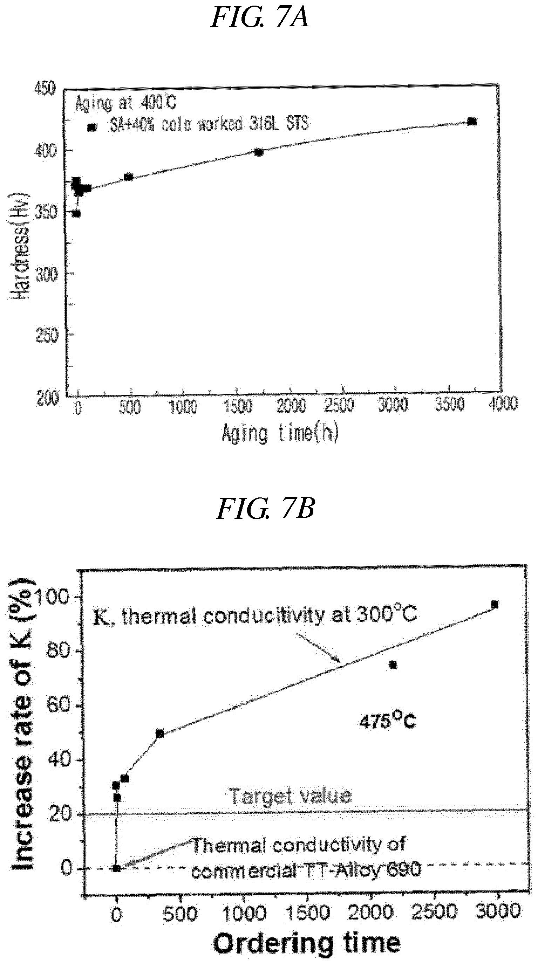

The amount of entropy decrease described above may be determined primarily by an amount of exothermic or endothermic heat measured by a DSC, but may also be measured by changes in the above-described physical properties. Since the entropy decrease may harden the structure like the baguette bread, the amount of entropy decrease may be determined by measuring a change in its hardness instead of the amount of exothermic or endothermic heat. Since the entropy decrease affects not only the hardness but also various physical properties of the structural material, it is possible to measure the amount of entropy decrease by tracking the changes in physical properties. Representative examples of the changes in physical properties due to an entropy decrease are the changes in hardness and thermal conductivity. When a 316L stainless steel and an Alloy 690 steam generator tube are aged for a long time at 400°C and 475°C, respectively, as shown in FIGS. 7A and 7B, the hardness of the 316L stainless steel and the thermal conductivity of the Alloy 690 increased, respectively.

FIG. 7A is a diagram showing an increase in hardness of the 316 stainless steel upon aging at 400 °C, and FIG. 7B is a diagram showing an increase in thermal conductivity of Alloy 690 upon aging at 475 °C.

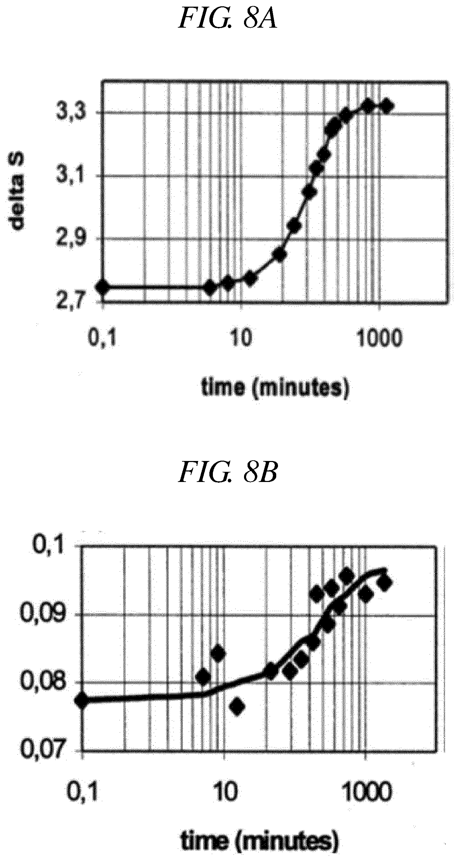

Referring to FIG. 7A and FIG. 7B, it may be seen that the hardness of 316L stainless steel increased gradually during aging at 400 °C, but the thermal conductivity of Alloy 690 increased rapidly up to 100 percent during aging at 475 °C. As is shown in FIG. 7A and FIG. 7B, the increases in the hardness and thermal conductivity during aging at high temperature are related to a compressive stress induced by an entropy decrease, which is the driving force to cause the formation of short-range order (SRO). Therefore, the larger the amount of entropy decrease, the greater the compressive stress generated, and the higher the volume of SRO, resulting in changes in physical properties such as the increases in hardness and simultaneously thermal conductivity. Therefore, considering the correlations between the changes in physical properties and the magnitude of compressive stress, and between the amount of entropy decrease and the magnitude of compressive stress, the amount of entropy decrease may be determined based on the changes in physical properties, or the magnitude of compressive stress may be determined based on the amount of entropy decrease. In addition to hardness and thermal conductivity, as shown in FIGS. 8A and 8B, the physical properties such as the thermoelectric power and the Barkuhausen noise amplitude may also be used.

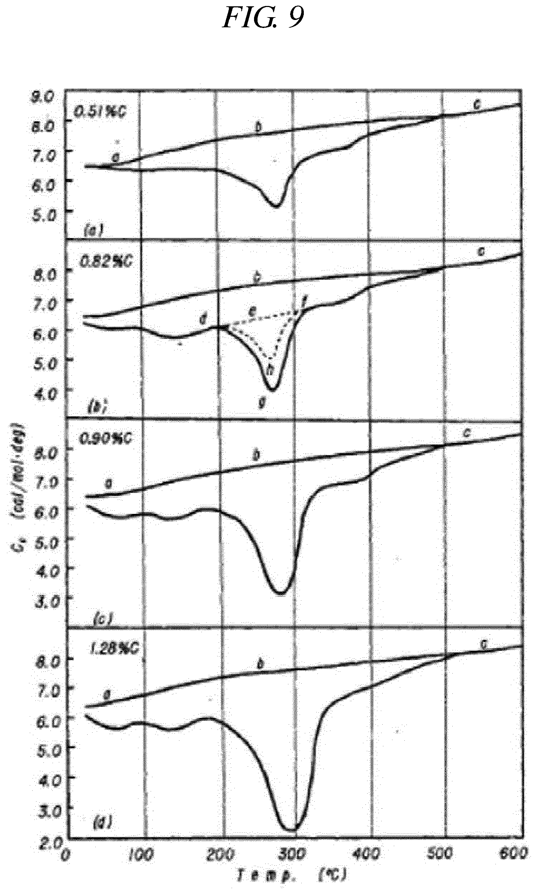

FIG. 8A and FIG. 8B are diagrams showing the thermoelectric power and the maximum amplitude of Barkuhausen rms signal, respectively, for a carbon steel containing 130 ppm carbon upon aging at 100°C, while FIG. 9 is a diagram showing the specific heats of a water-quenched carbon steel with carbon concentration after a homogenization treatment in the austenite region.

Referring to FIGS. 8A and 8B, it may be seen that the thermoelectric power and the maximum amplitude of Barkuhausen rms signal increased with the aging time when the carbon steel containing 130 ppm carbon was aged at 100 °C. As such, the increases in the thermoelectric power and the maximum amplitude of Barkuhausen rms signal during aging treatment at 100°C may be attributed to the entropy decrease of the carbon steel arising from an exothermic heat of the carbon steel during aging at 100 °C, as shown in FIGS. 9.

FIG. 9 is a graph showing the specific heats of a water-quenched carbon steel with carbon concentration after a homogenization treatment in an austenite region.

Referring to FIG. 9, it showed that the higher the carbon concentration of the carbon steel, the higher the amount of its exothermic heat at 100 °C. Therefore, with increasing carbon concentration, the amount of entropy decrease of the carbon steel increases due to a higher amount of exothermic heat, likely leading to a remarkable increase in the thermoelectric power and the maximum amplitude of Barkuhausen rms signal, respectively, shown in FIGS. 8A and 8B. Therefore, the amount of entropy decrease of the carbon steel can be determined from the amount of increases in the thermoelectric power and the maximum amplitude of Barkuhausen noise rms signal, leading to diagnosing the lifetime of the carbon steel. In particular, the results of FIGS. 8A, 8B, and 9 may be very effectively used in diagnosing the lifetime of pressure vessel steel and carbon steel pipes in nuclear power plants.

In one embodiment, since the electrical resistance of a material also changes according to the entropy decrease, the change in the electrical resistance may be measured to determine the entropy decrease.

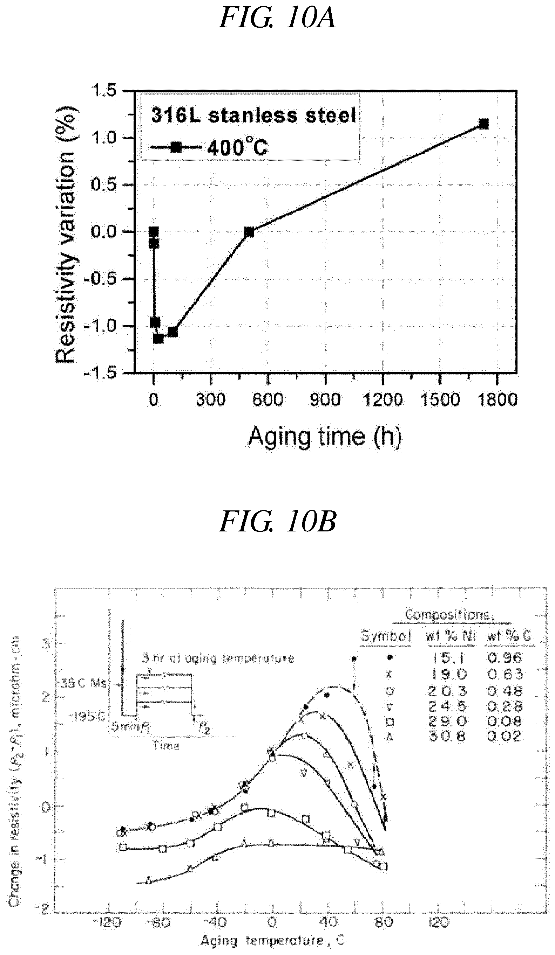

FIG. 10A is a graph showing a change in the electrical resistance of 40% cold-worked 316L stainless steel with aging time at 400 °C. FIG. 10B is a diagram showing changes in the electrical resistance of a Fe-Ni-C iron-based alloy with temperature upon isochronal aging for 3 hours. Referring to FIG. 10A, it may be seen that as the aging time at 400°C increased, the electrical resistance increased. Referring to FIG. 10B, it may be seen that with increasing concentration of carbon over nickel, the changes in electrical resistance increased. It may be seen that the increase in electrical resistance corresponds to an increase in the compressive stress caused by the entropy decrease. This is because when the compressive stress increases considerably due to a significant amount of entropy decrease, local tensile stresses generated in the vicinity of grain boundaries increase, causing an increase in the electrical resistance. Therefore, based on a change in the electrical resistance of the material, it is also possible to determine the amount of entropy decrease.

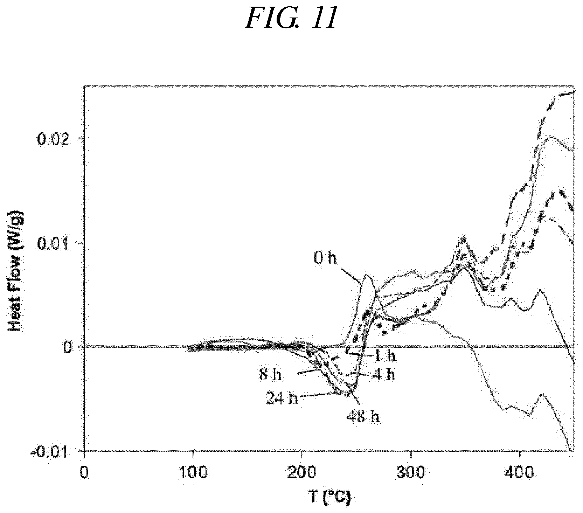

FIG. 11 is a diagram showing the DSC thermograms of Al-3.2Mg-0.18Cu alloy subjected to aging treatments after a solution treatment. Specifically, it is the diagram showing the results which were obtained after the Al-3.2Mg-0.18Cu alloy was solution-treated at about 550 °C for about 30 minutes, water-quenched, and then aged at about 180 °C for 0, 1, 4, 8, 24, 48 h, respectively, and finally was subjected to DSC tests.

Referring to FIG. 11, the unaged Al-3.2Mg-0.18Cu alloy showed no endothermic heat in the temperature range of 200 to 300°C except an exothermic heat at a temperature of about 250 °C or higher. However, the aged Al-3.2Mg-0.18Cu alloy at 180 °C showed only endothermic heat in that temperature range of 200 °C to 300 °C. The amount of endothermic heat was the highest in the Al-3.2Mg-0.18Cu alloy aged for 8 h at 180 °C, and the shear modulus of the Al-3.2Mg-0.18Cu alloy increased in proportion to the amount of endothermic heat. So, the increase in the amount of endothermic heat of the Al-3.2Mg-0.18Cu alloy with aging time means that the amount of entropy decrease of the Al-3.2Mg-0.18Cu alloy increased with aging time, triggering an increase in the shear modulus of the Al-3.2Mg-0.18Cu alloy in proportion to the amount of entropy decrease. Therefore, the amount of entropy decrease of the structure may also be determined through the measurements of the shear modulus or the elastic modulus (or Young's modulus) of the structure instead of the amount of exothermic or endothermic heat, which will be described below. Since the entropy decrease induces a compressive stress inside the structure and increases the bond strength of atoms in the structure, the shear modulus or elastic modulus of the structure will increase in proportion to the magnitude of compressive stress. This explains why the shear modulus and elastic modulus of the structure increase in proportion to the amount of endothermic heat.

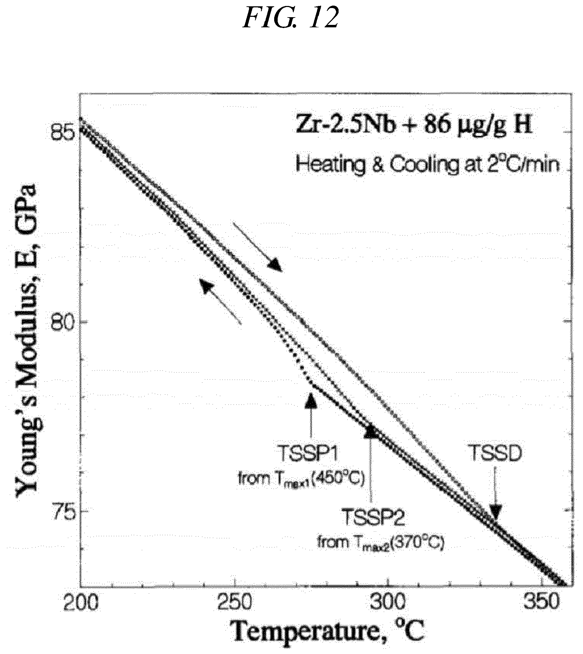

FIG. 12 is a diagram showing changes in the elastic modulus of a Zr-2.5Nb specimen containing 88 ppm hydrogen at the time of hydride dissolution and precipitation upon heating and cooling, respectively.

Referring to FIG. 12, upon hydride precipitation, an elastic modulus of the Zr-2.5Nb specimen rapidly increased while, upon hydride dissolution, the elastic modulus suddenly decreased. Such sudden changes in the elastic modulus have been used to determine the terminal solid solubility temperatures of hydrides for not only precipitation but also dissolution. An important thing is the fact that the elastic modulus showed a rapid increase upon hydride precipitation but a sudden drop upon hydride dissolution.

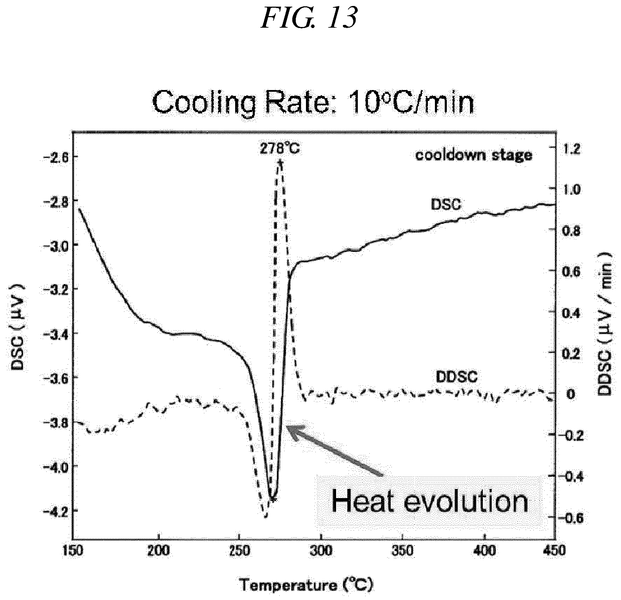

FIG. 13 is a diagram showing the specific heat of Zircaloy-2 having 106 ppm hydrogen upon cooling at a rate of 10 °C/min.

Referring to FIG. 13, since hydride precipitation involved an exothermic heat, the hydride precipitation means an entropy decrease. Thus, it means that the entropy decrease causes a compressive stress, triggering the precipitation of hydrides. With increasing amount of entropy decrease, the compressive stress generated in the zirconium matrix increases, leading to an increase in the elastic modulus of the zirconium matrix. Therefore, the amount of entropy decrease of the structure may be determined by tracking the changes in the elastic modulus of the structure. The elastic modulus of a structure may be measured with an APUCOT (Automatic Piezoelectric Ultrasonic Composite Oscillator Technique) equipment, or by various methods such as resonant ultrasound spectroscopy, acoustic resonance, ultrasound technique and nano-indentation.

As described above, according to the present invention, the lifetime of a structure may not be determined by external factors, but by an internal factor such as the magnitude of compressive stress caused by an entropy decrease. Judging from this fact, it may be predicted that if the compressive stress caused by the entropy decrease exceeds a certain limit value, the cracking initiates in the structure, leading to its failure. Therefore, the lifetime of the structure may be defined as the time of crack initiation or as the time to its failure. In one embodiment, the time of crack initiation may be considered as the lifetime of the structure.

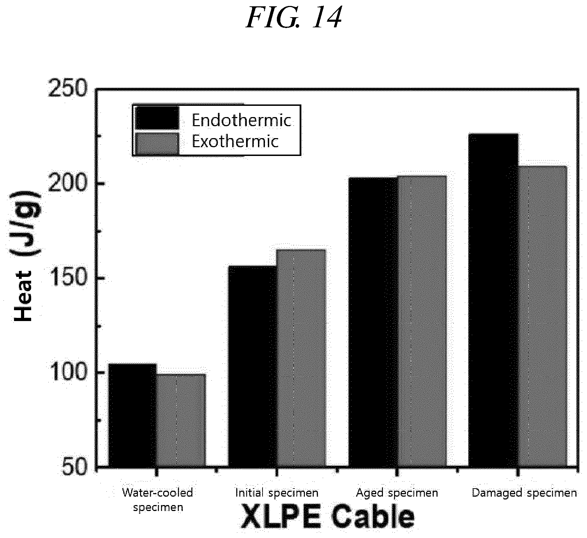

In the above <Equation 5> and <Equation 6>, a typical example in which an amount of exothermic or endothermic heat increases with increasing operating time, leading to an increase in the magnitude of compressive stress accumulated in the structure may be illustrated in FIG. 14, which showed an increase in the amounts of exothermic and endothermic heat with the degree of aging of a cross-linked polyethylene (XLPE) specimen widely being used as cable insulation material in nuclear power plants.

FIG. 14 is a diagram comparing the amounts of endothermic and exothermic heat with the degree of aging of a XLPE (cross-linked polyethylene) specimen widely used as cable insulation material, which were measured by DSC. Here, 4 different kinds of specimens are used: the as-receive (AR) specimen, the water quenched AR obtained from heating the AR specimen to 153°C and holding there for 30 minutes followed by water quenching, the aged AR specimen obtained from aging the AR specimen at 90°C for a long time, and the failed specimen obtained from breaking the aged AR specimen by tensile tests.

Referring to FIG. 14, the aged specimen and the failed specimen showed higher amounts of exothermic and endothermic heat than the AR specimen and the water quenched AR specimen. Also, since the change in the amount of heat means the change in entropy, the fact that the amount of exothermic and endothermic heat increased with the degree of aging means an increase in the amount of entropy decrease, leading to an increase in the compressive stress accumulated in the XLPE specimen.

Therefore, the lifetime of a structure may be determined by determining the constant k from an amount of heat of the structure measured at an arbitrary time and before an operation; and by directly measuring an amount of heat stored in the structure at the end of life, and by determining the peak temperature where the maximum amount of heat is released, the operating temperature, T, and an activation energy of entropy decrease, $Q_{\Delta S}$. Therefore, the lifetime of a structure may be determined regardless of types of structures and materials including ceramics, metals, plastics, or concretes, as long as the measured values of the parameters mentioned above are available.

Experiment 4:

In order to verify the method of diagnosing the lifetime of a structure according to the present invention, the expected lifetime of Alloy 600 based on Equation 5 or Equation 6 was compared with the measured lifetime of Alloy 600 components in nuclear power plants and in laboratory conditions. The operating conditions of nuclear power plants refer to the conditions exposed to the primary water cooling system of nuclear power plants (high temperature water in the range of 300 °C to 340 °C with 15 MPa pressure and pH of 7, but added with boron and lithium), and the laboratory conditions refers to the conditions exposed to high temperature water simulating the operating conditions of nuclear power plants.

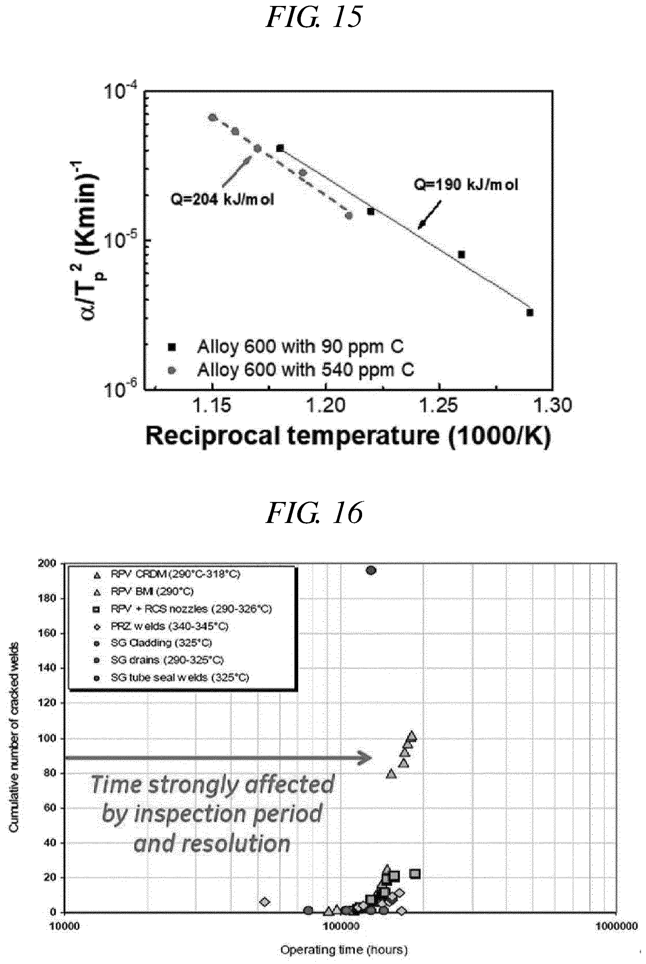

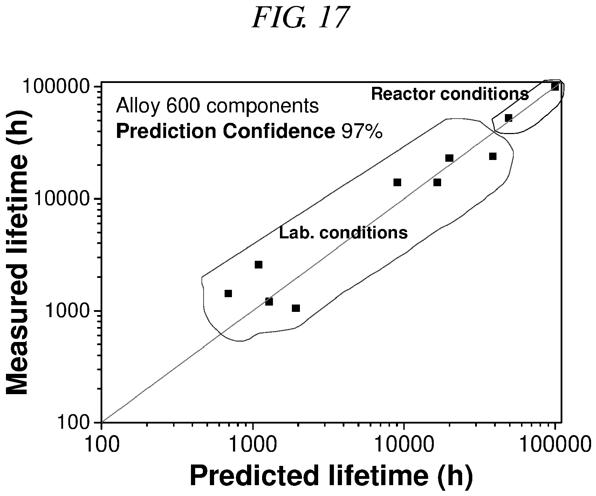

Here, it is assumed that a ratio of the amount of exothermic or endothermic heat at the end of life over the initial amount of exothermic or endothermic heat before an operation, $H_L/H_i$ is the same for all the Alloy 600 materials used. As the activation energy for entropy decrease of the Alloy 600 was found to change from 190 kJ/mol to 204 kJ/mol with carbon concentration, as shown in FIG. 15, these values were used as the activation energy of entropy decrease for Alloy 600. The minimum life time is the time defined when the $Q_{\Delta S}$ is equal to 190 kJ/mol, and the maximum life time corresponds to the $Q_{\Delta S}$ of 204 kJ/mol. The $T_p$ value, according to FIG. 4, corresponds to 793 K (or 520 °C), which was input into Equation 6. T is the reactor operation temperature or SCC test temperature, which was input into Equation 6. The constant k value was finally adjusted to 46 to predict the 6 to 11.4-year lifetime of the Alloy 600 components because the lifetime of the Alloy 600 components used in the operating conditions of nuclear power plants is reported to be in the range of 6 ~ 11.4 years, as shown in FIG. 16. As shown below in Table 2, the predicted value of the Alloy 600 lifetime determined by Equation 6 ranges from 5.6 years to 11.4 years, depending on the values of the activation energy for entropy decrease, $Q_{\Delta S}$. Assuming that the k-value determined in this way is effective even in the laboratory conditions, the predicted lifetime with the same values of k (= 46), Tp and $Q_{\Delta S}$ was compared with the measured lifetime time determined from the SCC tests performed in the various laboratory conditions that simulated the operating conditions of nuclear power plants. As shown in Table 2, the predicted lifetime by the present invention very precisely agrees with the SCC initiation time determined in the laboratory conditions. However, the measured lifetime at 332 °C was not the initiation time but the time to SCC failure. A comparison of the predicted and measured lifetime of the Alloy 600 is shown in FIG. 17 which is plotted based on the data of Table 2. The predicted lifetime of the Alloy 600 determined by the present invention almost perfectly agrees with the measured lifetime of Alloy 600 obtained both from the SCC tests in the laboratory conditions and from the SCC failures of Alloy 600 components operating in nuclear power plants. In particular, note that the Alloy 600 components used in the reactor operation conditions and the Alloy 600 specimens used in the SCC tests under the laboratory conditions must have been different from the Alloy 600 used to determine the parameters of the above Equation 5 or Equation 6 in view of the carbon concentration, the homogenization temperature, the cooling rate during cooling after a homogenization treatment and so on. Nevertheless, a very close agreement between the predicted lifetime by the present invention and the measured lifetime, as shown in Fig. 17, is very surprising. In conclusion, only if the structure-inherent parameters such as the activation energy for entropy decrease ($Q_{\Delta S}$), the peak temperature where the maximum amount of heat is released ($T_P$), and the operating temperature (T), and the structure-related constant (k) are provided, the method of diagnosing the lifetime of a structure of the present invention as defined by Equation 5 or Equation 6 may diagnose the lifetime of the target structure with high reliability, regardless of the types of structures.

Table 2 below is a table comparing the predicted lifetime of Alloy 600 according to an embodiment of the present invention and the crack initiation time determined during SCC tests in the laboratory conditions or the SCC initiation time determined in the reactor operating conditions of nuclear power plants.

[Table 2]

| Temperature (°C) | Operating Conditions | Alloy 600 | | REMARK |
|---|---|---|---|---|
| | | Expected Lifetime | Measured Lifetime | |
| 320 | Reactor Operating Conditions | 5.6-11.4y | 6-11.4y | SCC initiation time |
| 332 | Laboratory Conditions | 20,031-38,750 h | 23,932h (100% time to failure) | Time to failure (the time to crack initiation is 9487-11520h |
| 343 | Laboratory Conditions | 9,045-16,648 h | 13926 h | SCC initiation time |
| 365 | Laboratory Conditions | 1,930-12,181 h | 1050-12000 h | SCC initiation time |
| 380 | Laboratory Conditions | 691-1,090 h | 1416-2578 h | SCC initiation time |

The present invention described above is not limited to the above-described embodiments and the accompanying drawings, and it will be apparent to those skilled in the art that various substitutions, modifications and changes are possible within the scope of the technical spirit of the present invention.

**Claims**

1. A method of diagnosing the lifetime of a structure comprising;
   a step for preparing a structure to be measured;
   a step for measuring an amount of exothermic or endothermic heat of the structure;
   a step for determining an amount of entropy decrease of the structure from the measured amount of exothermic or endothermic heat; and
   a step for diagnosing the remaining lifetime of the structure from the measured amount of entropy decrease.

2. The method of diagnosing the lifetime of the structure of the claim 1, wherein the step for diagnosing the remaining lifetime of the structure utilizes a relationship between the amount of entropy decrease or the amount of exothermic or endothermic heat at the end of life and the amount of entropy decrease or the amount of exothermic or endothermic heat at an arbitrary time.

3. The method of diagnosing the lifetime of the structure of the claim 2, wherein the relationship is defined as $t_L=k(H_L/Hi)[(T_P-T)/T_P]^2exp[+Q_{\Delta S}/R(1/T-1/T_P)]$,
wherein, k is an intrinsic constant, R is the gas constant, Tp is the peak temperature where the maximum amount of heat is released, $Q_{\Delta S}$ is an activation energy for entropy decrease, T is an operating temperature, $H_L$ is an amount of exothermic or endothermic heat at the end of life, and $H_i$ is an amount of exothermic or endothermic heat at an arbitrary time.

4. The method of diagnosing the lifetime of the structure of the claim 3, wherein, the activation energy for entropy decrease $Q_{\Delta S}$ is determined by the Kissinger method where the peak temperature where the maximum amount of heat is released is plotted as a function of heating rate.

5. The method of diagnosing the lifetime of the structure of the claim 1, further comprising,
a step for measuring an actual compressive stress value of the structure; and
a step for correcting the predicted remaining lifetime based on the difference between the measured compressive stress value, and the predicted compressive stress value obtaining from the measured amount of exothermic or endothermic heat.

6. The method of diagnosing the lifetime of the structure of the claim 1, wherein measurements of the amount of exothermic or endothermic heat are performed by Differential Scanning calorimetry (DSC), Differential Thermal Analysis (DTA), Thermogravimetric Analysis (TGA), Thermomechanical Analysis (TMA), Dynamic Mechanical Analysis (DMA), or a combination thereof.

7. A method of diagnosing the lifetime of a structure comprising;
a step for preparing a structure to be measured;
a step for measuring the magnitude of compressive stress of the structure;
a step for measuring an amount of entropy decrease of the structure by using the measured magnitude of compressive stress; and
a step for diagnosing the remaining lifetime of the structure from the measured amount of entropy decrease.

8. The method of diagnosing the lifetime of the structure of the claim 7, wherein the step for diagnosing the remaining lifetime of the structure from the measured amount of entropy decrease utilizes the relationship between the amount of entropy decrease or the magnitude of compressive stress at the end of life of the structure and the amount of entropy decrease or the magnitude of compressive stress of the structure at an arbitrary time, which is defined as below:

$$t_L=k(\sigma_L/\sigma_i)[(T_P-T)/T_P]^2exp[+Q_{\Delta S}/R*(1/T-1/T_P)],$$

wherein, k is an intrinsic constant, R is the gas constant, Tp is the peak temperature of exothermic heat, $Q_{\Delta S}$ is an activation energy for entropy decrease, T is an operating temperature, $\sigma_L$ is a magnitude of compressive stress at the end of life, and $\sigma_i$ is a magnitude of compressive stress at an arbitrary time.

9. A method of diagnosing the lifetime of a structure comprising;
a step for preparing a structure to be measured;
a step for measuring an amount of lattice contraction of the structure;
a step for measuring an amount of entropy decrease of the structure by using the measured amount of lattice contraction; and
a step for diagnosing the remaining lifetime of the structure from the measured amount of entropy decrease.

10. The method of diagnosing the lifetime of the structure of the claim 9, wherein the step for diagnosing the remaining lifetime of the structure from the measured amount of entropy decrease utilizes the relationship between the amount of entropy decrease or the amount of lattice contraction at the end of life of the structure and the amount of entropy decrease or the amount of lattice contraction of the structure at an arbitrary time, which is defined as:

$$t_L = k(\Delta a_L/\Delta a_i)[(T_P-T)/T_P]^2 \exp[+Q_{\Delta S}/R*(1/T-1/T_P)],$$

wherein, k is an intrinsic constant, R is the gas constant, Tp is the peak temperature where the maximum amount of heat is released, $Q_{\Delta S}$ is an activation energy for entropy decrease, T is an operating temperature, $\Delta a_L$ is an amount of lattice contraction of the structure at the end of life, and $\Delta a_i$ is an amount of lattice contraction of the structure at an arbitrary time.

11. A method of diagnosing the lifetime of a structure comprising;
a step for preparing a structure to be measured;
a step for measuring the physical properties of the structure;
a step for measuring an amount of entropy decrease of the structure from the measured changes in physical properties; and
a step for diagnosing the remaining lifetime of the structure from the measured amount of entropy decrease,
wherein the physical properties include hardness, thermoelectric power, electrical resistance, Barkuhausen noise amplitude, shear modulus, elastic modulus or Young's modulus, or a combination thereof of the structure.

12. The method of diagnosing the lifetime of the structure of the claim 11, wherein the step for diagnosing the remaining lifetime of the structure from the measured amount of entropy decrease utilizes the relationship between the amount of entropy decrease or the amount of changes in physical properties at the end of life of the structure and the amount of entropy decrease or the amount of changes in physical properties at an arbitrary time, which is defined as:

$$t_L = k(\Delta \Pi_L/\Delta \Pi_i)[(T_P-T)/T_P]^2 \exp[+Q_{\Delta S}/R*(1/T-1/T_P)],$$

wherein, k is an intrinsic constant, R is the gas constant, Tp is the peak temperature where the maximum amount of heat is released, $Q_{\Delta S}$ is an activation energy for entropy decrease, T is an operating temperature, $\Delta \Pi_L$ is an amount of changes in physical properties of the structure at the end of life, and $\Delta \Pi_i$ is an amount of changes in physical properties of the structure at an arbitrary time.

13. A system for diagnosing the lifetime of a structure comprising:

a measuring device which measures an amount of exothermic or endothermic heat of the structure, a magnitude of compressive stress of the structure, an amount of lattice contraction of the structure, or the physical properties of the structure; and
an electronic device for measuring an amount of entropy decrease of the structure by using the measured amount of exothermic or endothermic heat, the measured magnitude of compressive stress, the measured amount of lattice contraction, or the measured changes in physical properties provided from the measuring device, and diagnosing the remaining lifetime of the structure from the measured amount of entropy decrease, wherein the physical properties include hardness, thermoelectric power, electrical resistance, Barkuhausen noise amplitude, shear modulus, elastic modulus or Young's modulus, or a combination thereof of the structure.

14. The system for diagnosing the lifetime of the structure of the claim 13, wherein the electronic device uses relationships between entropy decrease, an amount of exothermic or endothermic heat, an amount of lattice contraction, an amount of changes in physical properties or a magnitude of compressive stress at the end of life of the structure, and an amount of entropy decrease, an amount of exothermic or endothermic heat, an amount of lattice contraction, an amount of changes in physical properties or a magnitude of compressive stress of the structure at an arbitrary time.

15. The system for diagnosing the lifetime of the structure of the claim 13, wherein the above relationships are defined as $t_L=k(H_L/Hi)[(T_P-T)/T_P]^2\exp[+Q_{\Delta S}/R(1/T-1/T_P)]$, $t_L=k(\sigma_L/\sigma_i)[(T_P-T)/T_P]^2\exp[+Q_{\Delta S}/R*(1/T-1/T_P)]$, $t_L=k(\Delta a_L/\Delta a_i)[(T_P-T)/T_P]^2\exp[+Q_{\Delta S}/R*(1/T-1/T_P)]$, or $t_L=k(\Delta \Pi_L/\Delta \Pi_i)[(T_P-T)/T_P]^2\exp[+Q_{\Delta S}/R*(1/T-1/T_P)]$,
wherein k is an intrinsic constant, R is the gas constant, Tp is the peak temperature where the maximum amount of heat is released, $Q_{\Delta S}$ is an activation energy for entropy decrease, T is an operating temperature, $H_L$ is an amount of exothermic or endothermic heat at the end of life of the structure, and $H_i$ is an amount of exothermic or endothermic heat at an arbitrary time, $\sigma_L$ is a magnitude of compressive stress at the end of life of the structure, $\sigma_i$ is a magnitude of compressive stress at an arbitrary time, $\Delta a_L$ is an amount of lattice contraction at the end of life of the structure, $\Delta a_i$ is an amount of lattice contraction of the structure at an arbitrary time, $\Delta \Pi_L$ is an amount of changes in physical properties of the structure at the end of life, and $\Delta \Pi_i$ is an amount of changes in physical properties of the structure at an arbitrary time.

16. The system for diagnosing the lifetime of the structure of the claim 13, wherein the activation energy for entropy decrease, $Q_{\Delta S}$ is determined by the Kissinger method where the peak temperature where the maximum amount of heat is evolved is plotted as a function of heating rate.

17. The system for diagnosing the lifetime of the structure of the claim 13, wherein the electronic device measures the actual compressive stress of the structure, and corrects the predicted remaining lifetime from a difference between the measured compressive stress value and the predicted compressive stress value obtaining from the measured amount of exothermic or endothermic heat.

# FIG. 1A

| | |
|---|---|
| Preparing a structure to be measured | S1 |
| ↓ | |
| Measuring an amount of exothermic heat or endothermic heat of the structure | S2 |
| ↓ | |
| Measuring an amount of entropy decrease of the structure from the measured amount of exothermic or endothermic heat | S3 |
| ↓ | |
| Diagnosing the remaining lifetime of the structure from the measured amount of entropy decrease | S4 |

## FIG. 1B

| Preparing a structure to be measured | S1 |

| Measuring changes in the physical properties of the structure | S2' |

| Measuring an amount of entropy decrease of the structure from the measured physical properties of the structure | S3 |

| Diagnosing the remaining lifetime of the structure from the measured amount of the entropy decrease | S4 |

## FIG. 2

```
                    ┌──────────────────┐
                    │ Measuring Device │
                    │      (200)       │
                    └──────────────────┘
                              │
                              ▼
┌──────────────┐    ┌──────────────────┐    ┌──────────────┐
│  Input unit  │───▶│    Processor     │───▶│ Output unit  │
│    (110)     │    │      (100)       │    │    (120)     │
└──────────────┘    └──────────────────┘    └──────────────┘
                              │
                              ▼
                    ┌──────────────────┐
                    │  Storage unit    │
                    │     (130)        │
                    └──────────────────┘
```

## FIG. 3

*FIG. 4*

FIG. 5

## *FIG. 6A*

## *FIG. 6B*

## FIG. 7A

## FIG. 7B

*FIG. 8A*

*FIG. 8B*

## FIG. 9

## FIG. 10A

## FIG. 10B

*FIG. 11*

## FIG. 12

*FIG. 13*

*FIG. 14*

## FIG. 15

## FIG. 16

*FIG. 17*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200095746